# EUROPEAN PATENT SPECIFICATION

(11) **EP 4 237 018 B1**
(45) Date of publication and mention of the grant of the patent: **02.12.2026**
(21) Application number: 21814924.3
(22) Date of filing: 31.10.2021
(51) Int. Cl.: A61L 15/18, A61L 15/42

(54) **METHOD FOR REDUCING POST-OPERATIVE SCAR FORMATION USING MATERIALS COMPRISING COPPER, ZINC AND/OR SILVER COMPOUNDS APPLIED UNDER PERIPHERAL PRESSURE**
VERFAHREN ZUR REDUZIERUNG POSTOPERATIVER NARBENBILDUNG MITHILFE VON MATERIALIEN MIT KUPFER-, ZINK- UND/ODER SILBERVERBINDUNGEN, DIE UNTER PERIPHEREM DRUCK AUFGEBRACHT SIND
PROCÉDÉ DE RÉDUCTION DE FORMATION DE CICATRICE POST-OPÉRATOIRE À L'AIDE DE SUBSTANCES COMPORTANT DES COMPOSÉS DE CUIVRE, DE ZINC ET/OU D'ARGENT APPLIQUÉS SOUS PRESSION PÉRIPHÉRIQUE

(30) Priority: 02.11.2020 US 202063108495 P
(43) Date of publication of application: 06.09.2023
(73) Proprietor: Medcu Technologies Ltd., 4672211 Herzliya (IL)
(72) Inventor: LUSTIGER, Danny, 4626761 Herzliya (IL); BORKOW, Gadi, 7691000 Gibton (IL)
(74) Representative: Lavoix
(86) International application number: PCT/IL2021/051285
(87) International publication number: WO 2022/091102

(56) References cited:
- EP-A1- 3 305 335
- WO-A1-2019/012068

## Description

### TECHNICAL FIELD

The present application relates to the field of treating post-operative scar formation. In particular, the present application relates to the method for reducing post-operative scar formation on a skin using compositions or materials comprising copper, zinc and/or silver compounds, for example, copper oxide-impregnated wound dressings, applied to a surgical incision and surrounding inflamed tissue under peripheral pressure.

### BACKGROUND

Hypertrophic scarring often occurs following certain injuries such as burns, complex wounds and post-surgery wounds. Hypertrophic scars can be raised, abnormally pigmented and can cause itching or abnormal sensations in addition to the undesired aesthetic effect.

Transforming growth factor-β (TGF-β) is a family of growth factors involved in several processes of wound healing, such as inflammation, angiogenesis stimulation, fibroblast proliferation, collagen synthesis and deposition and remodelling of the new extracellular matrix. TGF-β promotes a rapid induction of fibrosis and angiogenesis *in vivo* and stimulation of collagen formation *in vitro.* TGF-β1 essentially improves wound healing and random flap survival in normal and irradiated rats.

Hypertrophic derived fibroblasts and hypertrophic scar tissue produced more mRNA and protein for TGF-β1 than normal skin or fibroblasts derived from normal skin, suggesting a possible role for TGF-β1 in hypertrophic scar formation (see R. Wang et al., "Hypertrophic scar tissues and fibroblasts produce more Transforming Growth Factor- β1 mRNA and protein than normal skin and cells", Wound Repair Regeneration 2000, Vol. 8, pp. 128-137). In hypertrophic scars, the expression of the TGF-β factor persists compared to normal wound healing where receptor expression decreases during the remodelling phase (see P. Schmidt, "Enhanced expression of Transforming Growth Factor-beta type 1 and type II receptors in wound granulation tissue and hypertrophic scar", American Journal of Pathology 1998, Vol. 152, pp. 485-493).

Copper is an essential trace element involved in many cellular, metabolic and physiological processes in almost all body tissues. N. Philips et al., in "Stimulation of cell proliferation and expression of matrixmetalloproteinase-1 and interluekin-8 genes in dermal fibroblasts by copper", Connective Tissue Research 2010, 51(3), pp. 224-229, demonstrated that copper is capable of stimulating dermal fibroblasts proliferation in skin. Further, N. Philips et al., "Beneficial regulation of fibrillar collagens, heat shock protein-47, elastin fiber components, Transforming Growth Factor-beta 1, vascular endothelial growth factor and oxidative stress effects by copper in dermal fibroblasts", Connective Tissue Research 2012, 53(5), pp. 373-378, showed that copper in the skin enhances production and secretion of different collagen and elastin types by fibroblasts.

EP 3305335 Al discloses materials for protecting and healing skin and wounds and mentions the addition of calcium and zinc-containing inorganic particles.

Also, it has been shown that copper is capable of stabilising the skin extracellular matrix once formed, serves as a cofactor of superoxide dismutase, an antioxidant enzyme present in the skin, important for protection against free radicals, serves as a cofactor of lysyl oxidase, an enzyme that catalyses lysine-derived crosslinks in the skin extracellular matrix, and inhibits cellular oxidative effects such as membrane damage and lipid peroxidation. However, the surprising combination effect of copper, zinc and/or silver containing material with absorbent, as well as compression capabilities of this material on reducing post-operative scar formation on a skin has not been known yet. This combination effect has now been unexpectedly discovered by the present inventors and constitutes the basis of the present invention.

### SUMMARY

The scope of the invention is defined in the appended claims.

The present application describes embodiments of a method for reducing post-operative scar formation on a skin of a patient comprising applying a material comprising copper, zinc and/or silver compounds to a postoperative wound under compression or under continuous peripheral pressure to the surgical incision and surrounding inflamed tissue, in order to reduce the inflammatory response, improve wound healing, reduce wounds complications and improve scar formation.

In some embodiments, said material is a woven or non-woven fabric, a foam, a knit fabric, or any type of fabric that is used to make wound dressings, plasters, gauze or the like. In one embodiment, this material is impregnated with about 0.1-10% w/w water-insoluble copper, zinc and/or silver particles or coated with about 0.1-10% w/w water-insoluble copper, zinc and/or silver particles or their composite particles. In another embodiment, this material is a conventional wound dressing, to which a composition comprising water-soluble copper, zinc and/or silver compounds or suspension of water-insoluble copper, zinc and/or silver compounds in water, in a solvent or in a mixture thereof, is applied. In some embodiments, the composition is in a form of a liquid, spray, gel, ointment or powder. In other embodiments, the composition comprises encapsulated copper, zinc and/or silver compounds applied to said conventional wound dressing.

In still other embodiments, said material is a negative pressure surgical wound dressing or a vacuum assisted closure wound dressing. In a certain embodiment, this negative pressure surgical wound dressing or the vacuum assisted closure wound dressing is impregnated with about 0.1-10% w/w water-insoluble copper, zinc and/or silver particles or coated with about 0.1-10% w/w water-insoluble copper, zinc and/or silver particles or their composite particles. In a particular embodiment, a composition comprising water-soluble copper, zinc and/or silver compounds or suspension of water-insoluble copper, zinc and/or silver compounds in water, in a solvent or in a mixture thereof, is applied to the negative pressure surgical wound dressing or the vacuum assisted closure wound dressing.

According to the invention, said water-insoluble copper particles are specifically cuprous iodide (CuI), cupric oxide (CuO) or cuprous oxide (Cu₂O) particles. The water-insoluble zinc particles are specifically zinc oxide (ZnO) particles. The water-insoluble silver particles are specifically silver chloride (AgCl) or silver oxide (Ag₂O). The water-insoluble composite particles are, for example, copper oxide with silver (CuO/Ag) or zinc oxide with silver (ZnO/Ag).

In still further embodiment, said composition is in a form of a liquid spray or ointment containing water-soluble copper, zinc and/or silver compounds. The exemplary water-soluble copper compounds of the embodiments are cupric sulphate (CuSO₄) and cupric chloride (CuCl₂). The exemplary water-soluble zinc compounds are zinc acetate (ZnOAc₂), zinc chloride (ZnCl₂) and zinc sulphate (ZnSO₄). The exemplary water-soluble silver compounds are silver fluoride (AgF) and silver nitrate (AgNO₃).

A non-limiting example of the material used in the present invention is a woven fabric impregnated or coated with approximately 0.1-10% w/w cuprous iodide (CuI) particles or a sterile wound dressing impregnated or coated with approximately 0.1-10% w/w Cu₂O particles. Another exemplary material of the present invention is a woven fabric which is impregnated or coated with about 0.1-10% w/w CuO/Ag nanocomposite particles or a sterile wound dressing impregnated or coated with approximately 0.1-10% w/w ZnO/Ag particles.

In another embodiment, said material is in a form of a polymeric film, fibre, filament or sheath. The exemplary polymeric film of the embodiments comprise polymers selected from the group of polyester, polypropylene, polyethylene, Nylon 66, Nylon 6, polyamide and polyurethane. The polymeric material of the present embodiment comprises water-insoluble particles of copper, zinc and/or silver compounds in a powdered form, embedded directly inside said film, fibre, filament or sheath.

Various embodiments may allow various benefits, and may be used in conjunction with various applications. The details of one or more embodiments are set forth in the accompanying figures and the description below. Other features, objects and advantages of the described techniques will be apparent from the description and drawings and from the claims

### BRIEF DESCRIPTION OF THE DRAWINGS

Disclosed embodiments will be understood and appreciated more fully from the following detailed description taken in conjunction with the appended figures. The drawings included and described herein are schematic and are not limiting the scope of the disclosure. It is also noted that in the drawings, the size of some elements may be exaggerated and, therefore, not drawn to scale for illustrative purposes. The dimensions and the relative dimensions do not necessarily correspond to actual reductions to practice of the disclosure.
**Figs. 1a** and **1b** show the reduction of TGF-β secretion by copper ions treated skin explants after wounding, and comparison between the TGF-β secretion by the untreated skin explants (naive control) and the burn skin explants (burn control) following wounding.
**Fig. 2** shows constant release of copper ions by copper-oxide impregnated dressing.
**Fig. 3** shows the skin condition seven weeks after bunion surgery treated with application of the copper-oxide containing wound dressings together with external compression post operatively.
**Fig. 4** shows an example of the post-operative reduction of swelling and scar visibility following the combination of peripheral pressure and application of the copper oxide impregnated wound dressing.
**Fig. 5** shows and example of the reduced inflammatory response after surgery following the combination of peripheral pressure and application of the copper oxide impregnated wound dressing of the present inventors, as visualised by significant reduced swelling and skin wrinkles.
**Fig. 6** shows and example of an enhanced closure of the gap formed in a scratch assay using 1 and 10 nM of copper ions eluted from the wound dressings of the present invention.

### DETAILED DESCRIPTION

In the following description, various aspects of the present application will be described. For purposes of explanation, specific configurations and details are set forth in order to provide a thorough understanding of the present application. However, it will also be apparent to one skilled in the art that the present application may be practiced without the specific details presented herein. Furthermore, well-known features may be omitted or simplified in order not to obscure the present application.

The term "comprising", used in the claims, is "open ended" and means the elements recited, or their equivalent in structure or function, plus any other element or elements which are not recited. It should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a material comprising x and z" should not be limited to materials consisting only of compounds x and z. Also, the scope of the expression "a method comprising the steps x and z" should not be limited to methods consisting only of these steps.

Unless specifically stated, as used herein, the term "about" is understood as within a range of normal tolerance in the art, for example within two standard deviations of the mean. In one embodiment, the term "about" means within 10% of the reported numerical value of the number with which it is being used, preferably within 5% of the reported numerical value. For example, the term "about" can be immediately understood as within 10%, 9%, 8%, 7%, 6%, 5%, 4%, 3%, 2%, 1%, 0.5%, 0.1%, 0.05%, or 0.01% of the stated value. In other embodiments, the term "about" can mean a higher tolerance of variation depending on for instance the experimental technique used. Said variations of a specified value are understood by the skilled person and are within the context of the present invention. As an illustration, a numerical range of "about 1 to about 5" should be interpreted to include not only the explicitly recited values of about 1 to about 5, but also include individual values and sub-ranges within the indicated range. Thus, included in this numerical range are individual values such as 2, 3, and 4 and sub-ranges, for example from 1-3, from 2-4, and from 3-5, as well as 1, 2, 3, 4, 5, or 6, individually. This same principle applies to ranges reciting only one numerical value as a minimum or a maximum. Unless otherwise clear from context, all numerical values provided herein are modified by the term "about". Other similar terms, such as "substantially", "generally", "up to" and the like are to be construed as modifying a term or value such that it is not an absolute. Such terms will be defined by the circumstances and the terms that they modify as those terms are understood by those of skilled in the art. This includes, at very least, the degree of expected experimental error, technical error and instrumental error for a given experiment, technique or an instrument used to measure a value.

As used herein, the term "and/or" includes any and all combinations of one or more of the associated listed items. Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

The present application discloses a method for reducing post-operative scar formation comprising applying a material comprising copper, zinc and/or silver compounds to a postoperative wound under continuous pressure to the surgical incision and surrounding inflamed tissue, in order to reduce the inflammatory response, improve wound healing, reduce wounds complications and improve scar formation.

In some embodiments, said material is a woven or non-woven fabric, a foam, a knit fabric, or any type of fabric that is used to make wound dressings, plasters, gauze or the like. In one embodiment, this material is impregnated with about 0.1-10% w/w water-insoluble copper, zinc and/or silver particles or coated with about 0.1-10% w/w water-insoluble copper, zinc and/or silver particles or their composite particles. In another embodiment, this material is a conventional wound dressing, to which a composition comprising water-soluble copper, zinc and/or silver compounds or suspension of water-insoluble copper, zinc and/or silver compounds in water, in a solvent or in a mixture thereof, is applied.

In some embodiments, the composition is in a form of a liquid, spray, gel, ointment or powder. In other embodiments, the composition comprises encapsulated copper, zinc and/or silver compounds applied to said conventional wound dressing.

In a further embodiment, said water-insoluble copper particles are specifically cuprous iodide (CuI), cupric oxide (CuO) or cuprous oxide (Cu₂O) particles. The water-insoluble zinc particles are specifically zinc oxide (ZnO) particles. The water-insoluble silver particles are specifically silver chloride (AgCl) or silver oxide (Ag₂O). The water-insoluble composite particles are, for example, copper oxide with silver (CuO/Ag) or zinc oxide with silver (ZnO/Ag).

In still further embodiment, said composition is in a form of a liquid spray or ointment containing water-soluble copper, zinc and/or silver compounds. The exemplary water-soluble copper compounds of the embodiments are cupric sulphate (CuSO₄) and cupric chloride (CuCl₂). The exemplary water-soluble zinc compounds are zinc acetate (ZnOAc₂), zinc chloride (ZnCl₂) and zinc sulphate (ZnSO₄). The exemplary water-soluble silver compounds are silver fluoride (AgF) and silver nitrate (AgNO₃).

A non-limiting example of the material used in the present invention is a woven fabric impregnated or coated with approximately 0.1-10% w/w cuprous iodide (CuI) particles or a sterile wound dressing impregnated or coated with approximately 0.1-10% w/w Cu₂O particles. Another exemplary material of the present invention is a woven fabric which is impregnated or coated with about 0.1-10% w/w CuO/Ag nanocomposite particles or a sterile wound dressing impregnated or coated with approximately 0.1-10% w/w ZnO/Ag particles.

In another embodiment, said material is in a form of a polymeric film, fibre, filament or sheath. The exemplary polymeric film of the embodiments comprise polymers selected from the group of polyester, polypropylene, polyethylene, Nylon 66, Nylon 6, polyamide and polyurethane. The polymeric material of the present embodiment comprises water-insoluble particles of copper, zinc and/or silver compounds in a powdered form, embedded directly inside said film, fibre, filament or sheath. The material being embedded or coated with the copper, zinc and/or silver compounds is capable of releasing Cu(I), Cu(II), Zn(II) and Ag(I) ions, or combination thereof upon contact with the damaged skin.

As mentioned in the *Background* section of the present invention, in hypertrophic scars, the expression of TGF-β persists compared to normal wound healing where receptor expression decreases during the remodeling phase. In *ex-vivo* skin explants studies, the present inventors have found that adding copper ions to the injured area immediately after burning drastically reduces the expression of TGF-β by the cells adjacent to the injured area indicating the potential reduction of scar formation *in vivo* following wounding by copper ions.

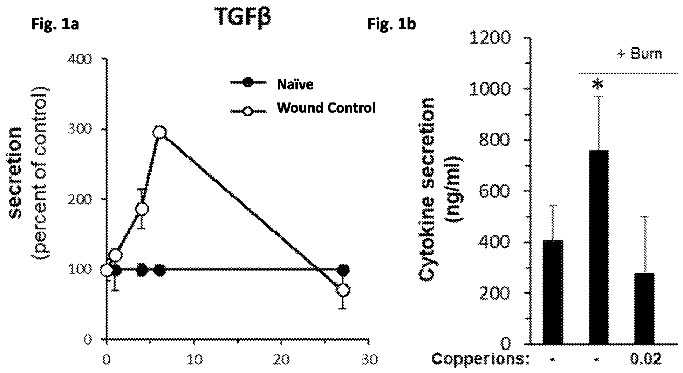

Reference is now made to **Figs. 1a** and **1b** showing the reduction of TGF-β secretion by copper ions treated skin explants after wounding, and also comparison between the TGF-β secretion by the untreated skin explants (naive control) and the burn skin explants (burn control) following wounding. In **Fig. 1a**, following burning, there is an approximately three-fold increase in the secretion of TGF-β as compared to the non-burned controls. In **Fig. 1b**, adding 0.02µM copper ions to the burned area prevented the TGF-β increased secretion by the tissue. In this figure, the star sign "*" indicates the statistically significant difference as compared to the naive control.

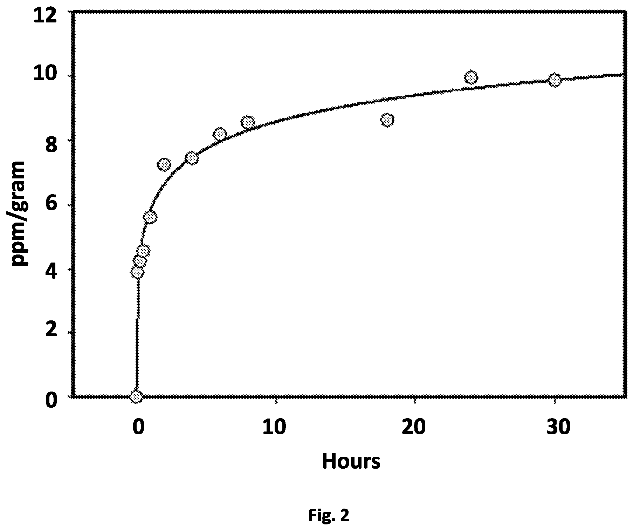

Wound dressings of the present inventors, containing copper, zinc and/or silver particles, serve as a reservoir of copper, zinc and/or silver slowly released in the presence of the skin humidity. Reference is now made to **Fig. 2** showing the constant secretion of copper ions by the copper oxide impregnated dressing of the present invention. This copper-oxide impregnated wound dressing that contains copper oxide microparticles serves as a reservoir of copper ions, small amounts (ppm) of which are slowly and constantly released in the presence of skin humidity and moisture in or around a postoperative wound exudate. **Fig. 2** demonstrates that 10 ppm copper ions are slowly and constantly released from the copper-oxide impregnated wound dressing in the presence of saline. In this experiment, 1-gram swatches of the copper-oxide impregnated wound dressings were incubated with saline at 37 °C between 5 minutes to 30 hours. The amount of copper ions eluting to the saline solution was determined by colorimetric assay using bicinchoninic acid (see A. J. Brenner and E. D. Harris in "A quantitative test for copper using bicinchoninic acid", Analytical Biochemistry 1995, Vol. 226(1), pp. 80-84).

Following surgery, a common post-operative condition is swelling (inflammation) of the operated area, especially around the opened skin and near a healing bone area, where surgical incision was made, and surrounding inflamed tissue. The swelling of the operated area stretches the skin and makes it harder for the smooth closure of the skin with new skin tissues, thus contributing to reduced healing capacity, which in turn may culminate in more noticeable scar and wound dehiscence.

Part of the inflammatory response that occurs after surgery is due to the penetration of microorganisms through the breach skin into the internal tissues. The presence of microorganisms in the wound stimulate the inflow of inflammatory cells into the wound, thereby enhancing the swelling of the operated area. The presence of microorganisms in the wound also results in increased exudates formation in the wound.

The presence of copper, zinc and/or silver in the vicinity of the post-operative surgical incision reduces the risk of dressing and wound microbial contamination. Reduction of microbial contamination of the wound reduces the inflammatory response and the influence of such responses on the scar formation.

Combining wound absorbent dressing impregnated or coated with copper, zinc and/or silver, with applying continuous pressure onto the surgical incision and surrounding inflamed tissue, significantly improve healing, reduce wounds complications and post-operative scar formation. Surgical wound blood and serous exudate are absorbed by such absorbent dressing. Reducing the presence of wound exudate reduces the substrate used by the bacteria for proliferating, and together with the potent biocidal effect of the copper, zinc and/or silver in the dressing, reduce the risk of surgical incision and wound site infection and dehiscence.

Applying compression (continuous pressure), which is also called "peripheral pressure", to the affected post-operative skin area is a practice commonly performed by medical practitioners and comprises applying elastic bandages on the affected skin area. While this is a simple and straightforward method to apply pressure to the whole circumference of the limb, it frequently obliterates lymphatic and venous return, causing distal swelling. Local pain from the elastic bandage may also occur.

Combining the desired effects of compression, absorbent and antimicrobial dressing into a single wound dressing can improve healing and reduce scar formation. This would also ease the surgeons' and nursing teams' task of applying and changing dressings, reduce dressing errors and improve overall healing. The wound dressing of the present invention therefore achieves three effects simultaneously: absorb the wound exudates, eliminate bacteria in the dressing and in the wound, and exert localised compression. In addition, due to the angiogenetic effect of copper, zinc and/or silver containing dressings, it will improve the blood circulation to the surgical incision, allowing reduction of local ischemic zones in the surgical incision site. The reduction of inflammatory cytokines will improve healing and the final stages of wound healing, *i.e.* reduce the scar formation.

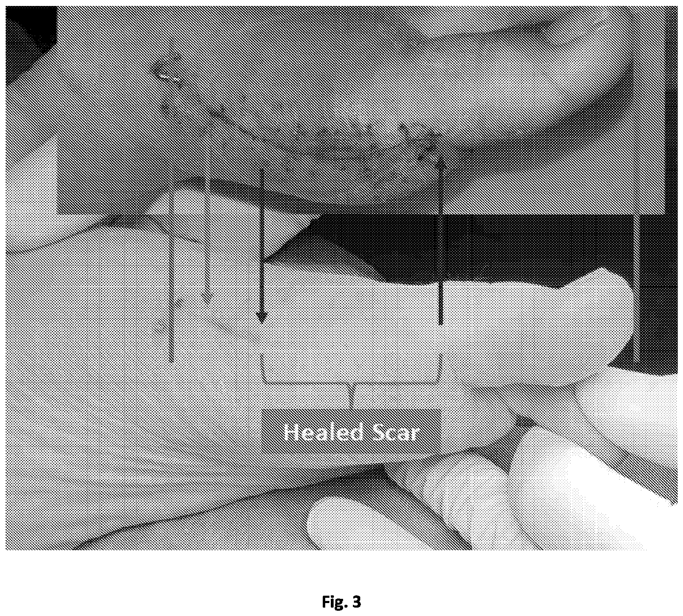

Indeed, as can be clearly seen in **Fig. 3****,** very significant reduction in the scar visibility was observed following the use of the copper oxide containing wound dressings and external compression post operatively. **Fig. 3** shows the skin condition seven weeks after bunion surgery treated with application of the copper-oxide containing wound dressing together with external compression post operatively. As can be seen, roughly 75-80% of the surgical incision has been healed completely and the scar is almost invisible. The rest of the incision demonstrates very delicate cicatrisation.

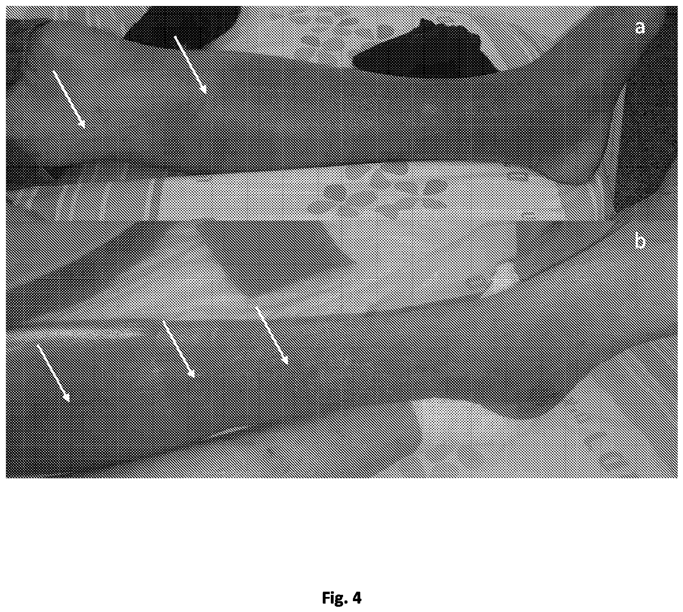

Reference is now made to **Fig. 4** showing an example of the post-operative reduction of swelling and scar visibility following the combination of peripheral pressure and application of the copper oxide impregnated wound dressing.

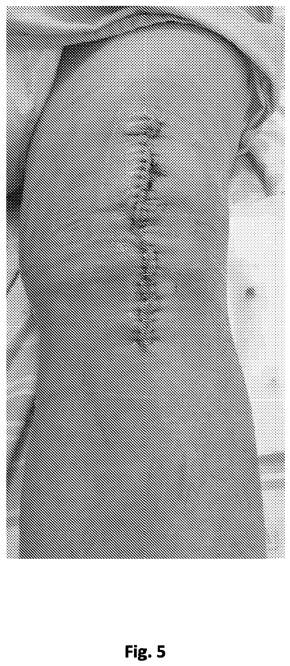

In addition, **Fig. 5** clearly demonstrates that no inflammation was observed post full knee surgery when using the wound dressing of the present inventors. The reduced inflammatory response following surgery, as visualised by significant reduced swelling and skin wrinkles, allows for faster wound healing and better scar formation.

"Postoperative wounds" are defined as wounds acquired during surgical procedures. Postoperative wound healing occurs after surgery and normally follows distinct bodily reactions: the inflammatory response, the proliferation of cellular and tissues that initiate healing, and the final remodelling. Postoperative wounds are different from other wounds in that they are anticipated and treatment is usually standardized depending on the type of surgery performed. Since the wounds are 'predicted', actions can be taken immediately after surgery that can reduce inflammations and promote healing. The body responds to postoperative wounds in the same manner as it does to tissue damage acquired in other circumstances. Inflammatory response is designed to create homeostasis, where the first stage is the inflammatory stage. The next stage of wound healing is the infiltration of leukocytes and release of cytokines into the tissue. Inflammatory response and the infiltration of leukocytes occur simultaneously. The final stage of postoperative wound healing is remodelling. Remodelling restores the structure of the tissue and that tissues ability to regain its function.

Negative-pressure wound therapy (NPWT) or a vacuum assisted closure (VAC) is a well-known therapeutic technique used for prophylaxis and preventing surgical site infections in wounds healing through primary closure. See, for example, Gill Norman (2020) et al. in "Negative pressure wound therapy for surgical wounds healing by primary closure", Cochrane Database of Systematic Reviews, 2020, 6(6), CD009261, DOI: 10.1002/14651858.CD009261.pub6. In some embodiments, the material applied in the method for reducing post-operative scar formation of the present invention is a negative pressure surgical wound dressing or a vacuum assisted closure wound dressing. It can be optionally used in combination with a suction pump and tubing to remove excess exudate and promote healing in acute or chronic wounds and second- and third-degree burns. In this case, instead of continuous pressure applied on the material of the present invention over a postoperative wound, the therapy involves the controlled application of sub-atmospheric pressure to the local wound environment using a sealed wound dressing connected to a vacuum pump. In one embodiment, this sealed wound dressing is impregnated with about 0.1-10% w/w water-insoluble copper, zinc and/or silver particles or coated with about 0.1-10% w/w water-insoluble copper, zinc and/or silver particles or their composite particles. In another embodiment, a composition comprising water-soluble copper, zinc and/or silver compounds or suspension of water-insoluble copper, zinc and/or silver compounds in water, in a solvent or in a mixture thereof, is applied to this sealed dressing upon applying the negative pressure to a surgical incision and surrounding inflamed tissue.

In another aspect, a method for reducing post-operative scar formation comprises: (a) applying a composition comprising copper, zinc and/or silver compounds to a surgical incision and surrounding inflamed tissue thereof, and (b) applying a negative-pressure surgical wound dressing or a vacuum-assisted closure wound dressing under negative or sub-atmospheric pressure to said surgical incision and surrounding inflamed tissue thereof. This results in reducing inflammatory response, improving wound healing, reducing wound complications, and thus, improving scar formation.

In some embodiments, steps (a) and (b) of the aforesaid method can be switched in order, so the first step would be applying the wound dressing, and the second step would be applying the composition. Also, in the aforesaid method, the negative-pressure surgical wound dressing or a vacuum-assisted closure wound dressing can be replaced with a conventional wound dressing readily available in any medical environment or in any medical or emergency kit, and that conventional wound dressing is applied under compression or under continuous peripheral pressure to a surgical incision and surrounding inflamed tissue, as described in the present invention.

In still other aspect, a method for removing excess exudate and promoting healing in acute or chronic wounds, or in second- or third-degree burns on a skin of a patient, comprises: (a) applying a composition comprising copper, zinc and/or silver compounds to an acute or chronic wound, or to a second- or third-degree burn, and surrounding inflamed tissue thereof, and (b) applying a negative-pressure surgical wound dressing or a vacuum-assisted closure wound dressing under negative or sub-atmospheric pressure to said acute or chronic wound, or to said second- or third-degree burn, and surrounding inflamed tissue thereof. In some embodiments of this method, steps (a) and (b) can be switched in order, so the first step would be applying the wound dressing, and the second step would be applying the composition. Also in this method, the negative-pressure surgical wound dressing or a vacuum-assisted closure wound dressing can be replaced with a conventional wound dressing readily available in any medical environment or in any medical or emergency kit, and that conventional wound dressing is applied under compression or under continuous peripheral pressure to a surgical incision and surrounding inflamed tissue, as described in the present invention.

Additional aspect of the present invention is a sealed wound dressing for use in negative-pressure wound therapy (NPWT) or a vacuum assisted closure (VAC), wherein said sealed wound dressing is impregnated with about 0.1-10% w/w water-insoluble copper, zinc and/or silver particles or coated with about 0.1-10% w/w water-insoluble copper, zinc and/or silver particles or their composite particles.

### Fast closure of human epidermal keratinocytes (but not of human fibroblasts) by copper ions

Hypertrophic scars occur when the body overproduces collagen as part of the extracellular matrix of the newly formed skin of a closing wound, which causes the scar to be raised above the surrounding skin. The cells responsible for the production of collagen are the dermal fibroblasts. At the end of the production of the extracellular layer, this layer is covered by the epidermal layer. When culturing human diploid fibroblasts or human epidermal keratinocytes *in vitro,* they form a confluent monolayer of cells.

Using the monolayers formed by those cell lines, a scratch assay can be performed in order to study the effect of different factors on the cell migration, mimicking cell migration during wound healing *in vivo* (see Chun-Chi Liang et al., "In vitro scratch assay: a convenient and inexpensive method for analysis of cell migration in vitro", Nature Protocols 2007, Vol. 2, pp. 329-333). The basic assay involves creating a "scratch" in a cell monolayer, capturing the images at the beginning and at regular intervals during cell migration to close the scratch. Following a scratch of the monolayer of human diploid fibroblasts (HDF cell line) and exposing them to different concentrations of ions secreted from the wound dressings of the present invention, no faster closure of the gap formed by the scratch was found.

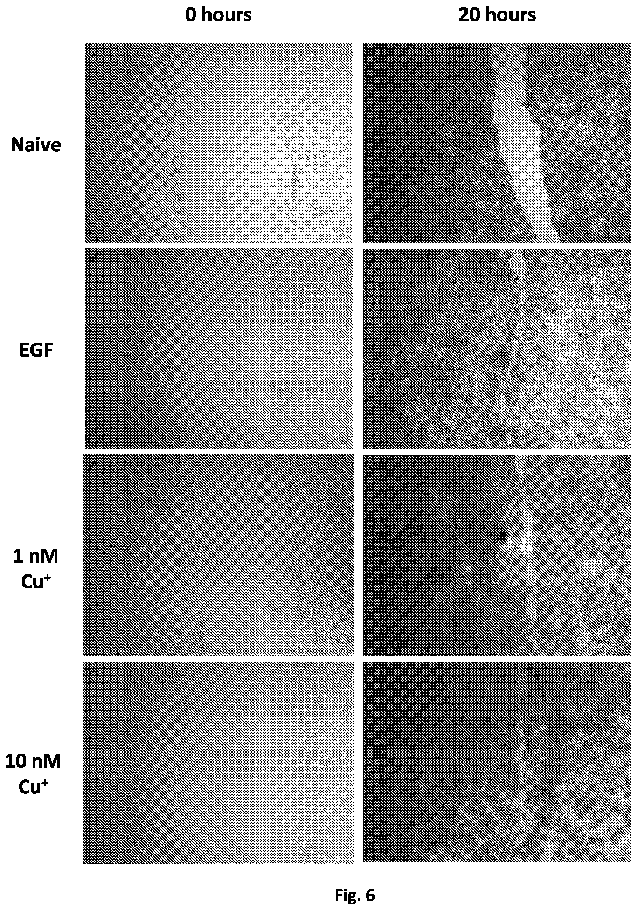

Surprisingly, as can be seen in **Fig. 6****,** when conducting a scratch assay using the human epidermal keratinocytes (HaCaT cell line) and exposing them to 1 nM or 10 nM of copper ions eluted from the wound dressings of the present invention, the extent of the closure of the cell gap was similar to that induced by the positive known control factor (Endothelial Growth Factor (EGF)).

The reduced scars and healed scars seen in **Figs. 3-5****,** in addition to the reduction in inflammation, can be also due to faster wound closure of the epidermal layer induced by the exposure of the wounds to the copper ions, without causing proliferation of the dermal fibroblasts, which cause the over-production of the collagen fibers responsible for the hypertrophic scars.

## Claims

1. A sealed wound dressing for negative-pressure wound therapy (NPWT) or a vacuum assisted closure (VAC), for removing excess exudate, promoting healing in acute or chronic wounds, or in second- or third-degree burns on a skin of a patient, and thereby reducing post-operative scar formation on a skin of a patient,
wherein said sealed wound dressing is impregnated with about 0.1-10% w/w water-insoluble copper and/or zinc particles, or coated with about 0.1-10% w/w water-insoluble copper and/or zinc particles,
wherein said water-insoluble copper particles are selected from cuprous iodide (CuI), cupric oxide (CuO), and cuprous oxide (Cu₂O), and said water-insoluble zinc particles are zinc oxide (ZnO).

2. The sealed wound dressing of claim 1, wherein a material for said sealed wound dressing is a woven fabric impregnated or coated with approximately 1-3% w/w CuI particles, or wherein said material is a sterile wound dressing impregnated or coated with approximately 1-3% w/w Cu₂O, CuO, ZnO or combinations thereof.

3. The sealed wound dressing of claims 1or 2, wherein a material for said sealed wound dressing is a sterile wound dressing impregnated or coated with approximately 1-3% w/w Cu₂O, CuO, ZnO or combinations thereof.

4. The sealed wound dressing of any one of claims 1 to 3, wherein a material for said sealed wound dressing is in a form of a polymeric film, fibre, filament, or sheath.

5. The sealed wound dressing of claim 4, wherein said polymeric film comprises polymers selected from polyester, polypropylene, polyethylene, Nylon 66, Nylon 6, polyamide and polyurethane.

6. The sealed wound dressing of claim 4, wherein the material for said sealed wound dressing comprises water-insoluble particles of copper and/or zinc compounds in a powdered form, embedded directly inside said film, fibre, filament or sheath, wherein a portion of said particles being exposed and protruding from the surface of the film, fibre, filament or sheath, or said material is coated with water-insoluble particles of copper and/or zinc for topical applications.

7. The sealed wound dressing of any one of claims 1 to 6, wherein said sealed wound dressing is connected to a vacuum pump via tubing.

## Patentansprüche

1. Ein abgedichteter Wundverband für die Unterdruck-Wundtherapie (NPWT) oder einen vakuumunterstützten Wundverschluss (VAC), zum Entfernen von überschüssigem Exsudat, zum Fördern der Heilung bei akuten oder chronischen Wunden, oder bei Verbrennungen zweiten oder dritten Grades auf der Haut eines Patienten, und dadurch zum Verringern der postoperativen Narbenbildung auf der Haut eines Patienten,
wobei der genannte abgedichtete Wundverband mit etwa 0,1-10 Gew.-% wasserunlöslichen Kupfer- und/oder Zinkpartikeln imprägniert ist oder mit etwa 0,1-10 Gew.-% wasserunlöslichen Kupfer- und/oder Zinkpartikeln beschichtet ist,
wobei die genannten wasserunlöslichen Kupferpartikel aus Kupfer(I)-iodid (CuI), Kupfer(II)-oxid (CuO) und Kupfer(I)-oxid (Cu₂O) ausgewählt sind und die genannten wasserunlöslichen Zinkpartikel Zinkoxid (ZnO) sind.

2. Der abgedichtete Wundverband nach Anspruch 1, wobei ein Material für den genannten abgedichteten Wundverband ein gewebter Stoff ist, der mit etwa 1-3 Gew.-% CuI-Partikeln imprägniert oder beschichtet ist, oder wobei das genannte Material eine sterile Wundauflage ist, die mit etwa 1-3 Gew.-% Cu₂O, CuO, ZnO oder Kombinationen davon imprägniert oder beschichtet ist.

3. Der abgedichtete Wundverband nach Ansprüchen 1 oder 2, wobei ein Material für den genannten abgedichteten Wundverband eine sterile Wundauflage ist, die mit etwa 1-3 Gew.-% Cu₂O, CuO, ZnO oder Kombinationen davon imprägniert oder beschichtet ist.

4. Der abgedichtete Wundverband nach einem der Ansprüche 1 bis 3, wobei ein Material für den genannten abgedichteten Wundverband in Form einer Polymerfolie, Faser, eines Filaments oder einer Hülle vorliegt.

5. Der abgedichtete Wundverband nach Anspruch 4, wobei die genannte Polymerfolie Polymere beinhaltet, die aus Polyester, Polypropylen, Polyethylen, Nylon 66, Nylon 6, Polyamid und Polyurethan ausgewählt sind.

6. Der abgedichtete Wundverband nach Anspruch 4, wobei das Material für den genannten abgedichteten Wundverband wasserunlösliche Partikel von Kupfer- und/oder Zinkverbindungen in Pulverform umfasst, die direkt innerhalb der genannten Folie, Faser, des genannten Filaments oder der genannten Hülle eingebettet sind, wobei ein Teil der genannten Partikel freiliegend und aus der Oberfläche der Folie, Faser, des Filaments oder der Hülle hervorstehend, oder wobei das genannte Material für topische Anwendungen mit wasserunlöslichen Partikeln aus Kupfer und/oder Zink beschichtet ist.

7. Abgedichteter Wundverband nach einem der Ansprüche 1 bis 6, wobei der genannte abgedichtete Wundverband über einen Schlauch mit einer Vakuumpumpe verbunden ist.

## Revendications

1. Un pansement étanche pour plaie destiné à une thérapie des plaies par pression négative (NPWT) ou à une fermeture assistée sous vide (VAC), pour éliminer l'exsudat en excès, favoriser la cicatrisation dans des plaies aiguës ou chroniques, ou dans des brûlures du deuxième ou du troisième degré sur une peau d'un patient, et réduire ainsi la formation de cicatrices post-opératoires sur une peau d'un patient,
dans lequel ledit pansement étanche pour plaie est imprégné d'environ 0,1-10 % p/p de particules insolubles dans l'eau de cuivre et/ou de zinc, ou revêtu d'environ 0,1-10 % p/p de particules insolubles dans l'eau de cuivre et/ou de zinc,
dans lequel lesdites particules de cuivre insolubles dans l'eau sont choisies parmi l'iodure cuivreux (CuI), l'oxyde cuivrique (CuO) et l'oxyde cuivreux (Cu₂O), et lesdites particules de zinc insolubles dans l'eau sont de l'oxyde de zinc (ZnO).

2. Le pansement étanche pour plaie selon la revendication 1, dans lequel un matériau pour ledit pansement étanche pour plaie est un tissu tissé imprégné ou revêtu d'environ 1-3 % p/p de particules de CuI, ou dans lequel ledit matériau est un pansement stérile pour plaie imprégné ou revêtu d'environ 1-3 % p/p de Cu₂O, CuO, ZnO ou de combinaisons de ceux-ci.

3. Le pansement étanche pour plaie selon les revendications 1 ou 2, dans lequel un matériau pour ledit pansement étanche pour plaie est un pansement stérile pour plaie imprégné ou revêtu d'environ 1-3 % p/p de Cu₂O, CuO, ZnO ou de combinaisons de ceux-ci.

4. Le pansement étanche pour plaie selon l'une quelconque des revendications 1 à 3, dans lequel un matériau pour ledit pansement étanche pour plaie est sous la forme d'un film polymère, d'une fibre, d'un filament ou d'une gaine.

5. Le pansement étanche pour plaie selon la revendication 4, dans lequel ledit film polymère comprend des polymères choisis parmi le polyester, le polypropylène, le polyéthylène, le Nylon 66, le Nylon 6, le polyamide et le polyuréthane.

6. Le pansement étanche pour plaie selon la revendication 4, dans lequel le matériau pour ledit pansement étanche pour plaie comprend des particules insolubles dans l'eau de composés de cuivre et/ou de zinc sous une forme pulvérulente, incorporées directement à l'intérieur dudit film, de ladite fibre, dudit filament ou de ladite gaine, dans lequel une partie desdites particules étant exposées et faisant saillie depuis la surface du film, de la fibre, du filament ou de la gaine, ou ledit matériau est revêtu de particules insolubles dans l'eau de cuivre et/ou de zinc destinées à des applications topiques.

7. Le pansement étanche pour plaie selon l'une quelconque des revendications 1 à 6, dans lequel ledit pansement étanche pour plaie est raccordé à une pompe à vide par l'intermédiaire d'une tubulure.
